# EUROPEAN PATENT APPLICATION

(11) **EP 2 077 526 A2**
(43) Date of publication of application: **08.07.2009**
(21) Application number: 09250003.2
(22) Date of filing: 02.01.2009
(51) Int. Cl.: G06T 7/00, A61B 8/14

(54) **Three-dimensional image reconstruction using doppler ultrasound**

(30) Priority: 04.01.2008 US 969504
(71) Applicant: Biosense Webster, Inc., Diamond Bar, CA 91765 (US)
(72) Inventor: Govari, Assaf, 34400 Haifa (IL); Altmann, Andres Claudio, 34614 Haifa (IL); Ephrath, Yaron, 35170 Karkur (IL); Schwartz, Yitzhack, 34606 Haifa (IL)
(74) Representative: Small, Gary James

(57) **Abstract**

A method for imaging of an anatomical structure includes acquiring a plurality of ultrasonic images of the anatomical structure. At least one of the images includes Doppler information. One or more contours of the anatomical structure are generated from the Doppler information. A three-dimensional image of the anatomical structure is reconstructed from the plurality of ultrasonic images, using the one or more contours.

## Description

### FIELD OF THE INVENTION

The present invention relates generally to imaging, and in particular to medical imaging.

### BACKGROUND OF THE INVENTION

Methods for 3-D mapping of the endocardium (i.e., the inner surfaces of the heart) are known in the art. For example, U.S. Patent 5,738,096 to Ben-Haim, which is assigned to the assignee of the present invention, and whose disclosure is incorporated herein by reference, describes a method for constructing a map of the heart. An invasive probe or catheter is brought into contact with multiple locations on the wall of the heart. The position of the invasive probe is determined for each location, and the positions are combined to form a structural map of at least a portion of the heart.

In some systems, such as the one described by U.S. Patent 5,738,096 cited above, additional physiological properties, as well as local electrical activity on the surface of the heart, are also acquired by the catheter. A corresponding map incorporates the acquired local information.

Some systems use hybrid catheters that incorporate position sensing. For example, U.S. Patent 6,690,963 to Ben-Haim et al., which is assigned to the assignee of the present invention, and whose disclosure is incorporated herein by reference, describes a locating system for determining the location and orientation of an invasive medical instrument.

U.S. Patent Application Publication No. 2006/0241445 by Altmann et al., which is assigned to the assignee of the present invention, and whose disclosure is incorporated herein by reference, describes a method for modeling an anatomical structure. A plurality of ultrasonic images of the anatomical structure is acquired using an ultrasonic sensor at different spatial positions. Location and orientation coordinates of the ultrasonic sensor are measured at each of these spatial positions. Contours-of-interest that refer to features of the anatomical structure are marked in one or more of the ultrasonic images. A three-dimensional (3-D) model of the anatomical structure is constructed, based on the contours-of-interest and on the measured location and orientation coordinates.

U.S. Patent 6,773,402 to Govari et al., which is assigned to the assignee of the present invention, and whose disclosure is incorporated herein by reference, describes a system for 3-D mapping and geometrical reconstruction of body cavities, particularly of the heart. The system uses a cardiac catheter comprising a plurality of acoustic transducers, which emit ultrasound waves that are reflected from the surface of the cavity and are received by the transducers. The distance from each of the transducers to a point or area on the surface opposite the transducer is determined, and the distance measurements are combined to reconstruct the 3-D shape of the surface. The catheter also comprises position sensors, which are used to determine location and orientation coordinates of the catheter within the heart. In one embodiment, the processing circuitry analyzes the frequency, as well as the time of flight, of the reflected waves in order to detect a Doppler shift. The Doppler measurement is used to determine and map the heart wall velocity.

U.S Patent 5,961,460, to Guracar et al., whose disclosure is incorporated herein by reference, describes an ultrasonic imaging system that generates Doppler and B-mode (two-dimensional diagnostic ultrasound) image signals, and then uses a modulated, non-linear mapping function to combine the Doppler and B-mode image signals into an output signal.

U.S. Patent 6,679,843, to Ma et al., whose disclosure is incorporated herein by reference, describes a method of reducing an elevation fold-in artifact by combining Doppler and B-mode image signals using a modulated, non-linear function. Portions of the B-mode image signal associated with stationary tissue are intact while portions of the B-mode image signal associated with flow are substantially suppressed.

### SUMMARY OF THE INVENTION

Three-dimensional (3-D) images of organs such as the heart are useful in many catheter-based diagnostic and therapeutic applications. Real-time imaging improves physician performance and enables even relatively inexperienced physicians to perform complex surgical procedures more easily. 3-D imaging also helps to reduce the time needed to perform some surgical procedures. Additionally, 3-D ultrasonic images may be used in planning complex procedures and catheter maneuvers.

To create a meaningful 3-D reconstruction from two-dimensional (2-D) ultrasound scans, the computer must know which features of the 2-D images represent actual contours of the organ of interest. A common solution to this problem in the prior art is for a user of the ultrasound imaging system to "help" the computer by tracing the contours on the 2-D image. This solution is used, for example, in U.S. Patent Application Publication No. 2006/0241445 cited above.

Some embodiments of the present invention use Doppler ultrasound to provide contour locations of the organ automatically or semi-automatically, wherein the user needs at most to review and possibly correct contours generated by the computer. In the case of the heart, for example, Doppler images clearly differentiate the interior volume of the heart from the heart walls due to the speed of blood flow within the heart. This phenomenon is particularly marked in the blood vessels leading into and out of the heart chambers.

Alternate embodiments of the present invention use Doppler ultrasound to determine locations of movement, typically of blood, but also of tissue. These locations may be used to reconstruct a 3-D model of regions of movement, such as blood flow and/or a surface bounding such regions, without forming or displaying contours of organs surrounding the regions.

There is therefore provided, according to an embodiment of the present invention a method for imaging an anatomical structure, including:

acquiring a plurality of ultrasonic images of the anatomical structure, at least one of the images comprising Doppler information;

generating one or more contours of the anatomical structure using the Doppler information; and

reconstructing a three-dimensional image of the anatomical structure from the plurality of ultrasonic images using the one or more contours.

Typically, generating the one or more contours includes determining a boundary between a first region of the anatomical structure having a speed of movement greater than or equal to a first value and a second region of the anatomical structure wherein the speed of movement is less than or equal to a second value smaller than the first value. The first value may be 0.08 m/s and the second value may be 0.03 m/s.

In one embodiment the anatomical structure includes a heart, and acquiring the plurality of ultrasonic images includes inserting a catheter including an ultrasonic sensor into a chamber of the heart and moving the catheter between a plurality of spatial positions within the chamber. Typically, the method also includes measuring location and orientation coordinates of the ultrasonic sensor, and synchronizing the plurality of ultrasonic images and the location and orientation coordinates relative to a synchronizing signal including one of an electrocardiogram (ECG) signal, an internally-generated synchronization signal and an externally-supplied synchronization signal.

The three-dimensional image may include a three-dimensional surface model of the anatomical structure, and the method may further include:

measuring at least one of a tissue characteristic, a temperature and a rate of flow of blood, synchronized to the synchronizing signal, to produce a parametric map; and

overlaying the parametric map on the three-dimensional surface model.

In a disclosed embodiment acquiring the plurality of ultrasonic images includes moving an ultrasonic sensor generating the ultrasonic images so that a velocity of movement of the ultrasonic sensor is less than a pre-determined threshold velocity.

Alternatively or additionally, acquiring the plurality of ultrasonic images includes determining a velocity of movement of an ultrasonic sensor generating the ultrasonic images, and correcting the Doppler information responsively to the velocity of movement.

The three-dimensional image may include a three-dimensional skeleton model of the anatomical structure and/or a three-dimensional surface model of the anatomical structure.

The method may include overlaying an electro-anatomical map on the three-dimensional surface model.

The method may include overlaying information imported from one or more of a Magnetic Resonance Imaging (MRI) system, a Computerized Tomography (CT) system and an x-ray imaging system on the three-dimensional surface model.

There is further provided, according to an embodiment of the present invention, a method for imaging an anatomical structure, including:

acquiring a plurality of two-dimensional Doppler images of elements moving in proximity to the anatomical structure; and

reconstructing a three-dimensional image of the moving elements.

Typically, reconstructing the three-dimensional image includes displaying the three-dimensional image absent the anatomical structure.

In one embodiment the method includes setting a threshold speed for the moving elements, and reconstructing the three-dimensional image includes displaying the moving elements having speeds greater than the threshold speed.

In a disclosed embodiment reconstructing the three-dimensional image includes determining a surface bounding at least some of the elements, and displaying the surface.

There is further provided, according to an embodiment of the present invention, a system for imaging an anatomical structure, including:

a probe, including an ultrasonic sensor, which is configured to acquire a plurality of ultrasonic images of the anatomical structure, at least one of the images including Doppler information; and

a processor, coupled to the ultrasonic sensor, which is configured to generate one or more contours of the anatomical structure using the Doppler information and to reconstruct a three-dimensional image of the anatomical structure from the plurality of ultrasonic images using the one or more contours.

There is further provided, according to an embodiment of the present invention, a system for imaging an anatomical structure, including:

a probe, including an ultrasonic sensor, which is configured to acquire a plurality of two-dimensional Doppler images of elements moving in proximity to the anatomical structure; and

a processor which is configured to reconstruct a three-dimensional image of the moving elements from the two-dimensional Doppler images.

There is further provided, according to an embodiment of the present invention a computer software product for imaging an anatomical structure, including a computer-readable medium in which computer program instructions are stored, which instructions, when read by a computer, cause the computer to acquire a plurality of ultrasonic images of the anatomical structure, at least one of the images including Doppler information, to generate one or more contours of the anatomical structure using the Doppler information, and to reconstruct a three-dimensional image of the anatomical structure from the plurality of ultrasonic images using the one or more contours.

### BRIEF DESCRIPTION OF THE DRAWINGS

For a better understanding of the present invention, reference is made to the detailed description of the invention, by way of example, which is to be read in conjunction with the following drawings, wherein like elements are given like reference numerals, and wherein:

Fig. 1 is a schematic, pictorial illustration of a system for cardiac mapping and imaging, in accordance with an embodiment of the present invention;

Fig. 2 is a schematic, pictorial illustration of a catheter, in accordance with an embodiment of the present invention;

Figs. 3-6 are schematic images of a non-human heart, in accordance with an embodiment of the present invention;

Fig. 7 is a 3-D skeleton model of the heart shown in Figs. 3-6, in accordance with an embodiment of the present invention;

Fig. 8 is a 3-D surface model of the heart shown in Figs. 3-6, in accordance with an embodiment of the present invention;

Fig. 9 is a flow chart that schematically illustrates a method for cardiac mapping and imaging, in accordance with an embodiment of the present invention;

Fig. 10 is a schematic image of a non-human heart, in accordance with an alternate embodiment of the present invention; and

Fig. 11 is a flow chart that schematically illustrates a method for cardiac mapping and imaging, in accordance with an alternate embodiment of the present invention.

### DETAILED DESCRIPTION OF THE INVENTION

In the following description, numerous specific details are set forth in order to provide a thorough understanding of the present invention. It will be apparent to one skilled in the art, however, that the present invention may be practiced without these specific details. In other instances, well-known circuits, control logic, and the details of computer program instructions for conventional algorithms and processes have not been shown in detail in order not to obscure the present invention unnecessarily.

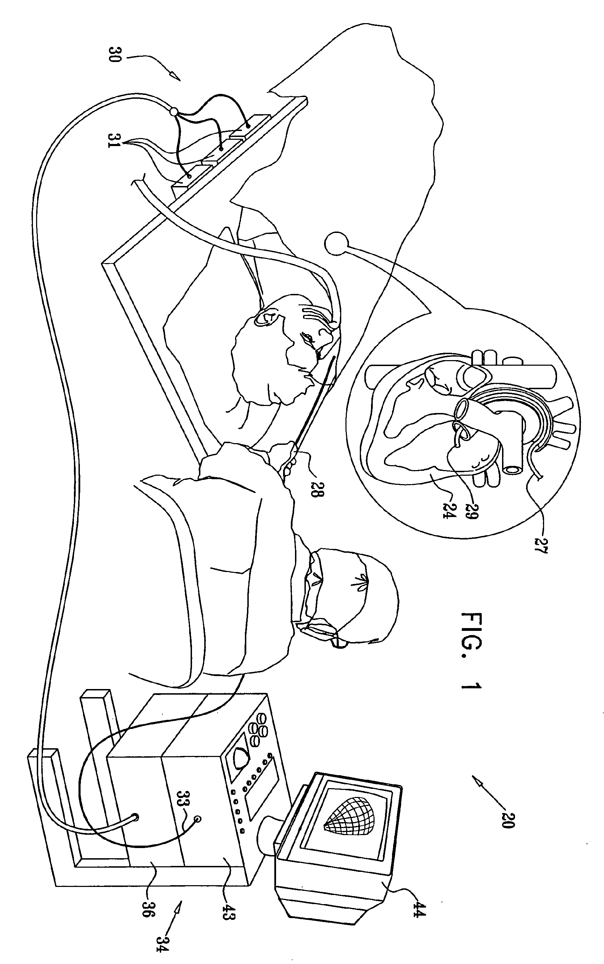

Turning now to the drawings, reference is initially made to Fig. 1, which is a schematic, pictorial illustration of a system 20 for mapping and imaging a heart 24 of a patient, in accordance with an embodiment of the present invention. System 20 comprises a probe, for example a catheter 27, which is inserted by a physician into a chamber of the heart through a vein or artery. Catheter 27 typically comprises a handle 28 for operation of the catheter by the physician. Suitable controls on handle 28 enable the physician to steer, locate and orient a distal end 29 of catheter 27 as desired.

System 20 comprises a positioning subsystem 30 that measures location and orientation coordinates of distal end 29 of catheter 27. In the specification and in the claims, the term "location" refers to the spatial coordinates of an object such as the distal end of the catheter, the term "orientation" refers to angular coordinates of the object, and the term "position" refers to the full positional information of the object, comprising both location and orientation coordinates.

In one embodiment, positioning subsystem 30 comprises a magnetic position tracking system that determines the position of distal end 29 of catheter 27. Positioning subsystem 30 generates magnetic fields in a predefined working volume in the vicinity of a patient, and senses these fields in a sensor, described below, in catheter 27. Positioning subsystem 30 typically comprises a set of external radiators, such as field generating coils 31, which are located in fixed, known positions external to the patient. Coils 31 generate fields, typically magnetic fields, in the vicinity of heart 24.

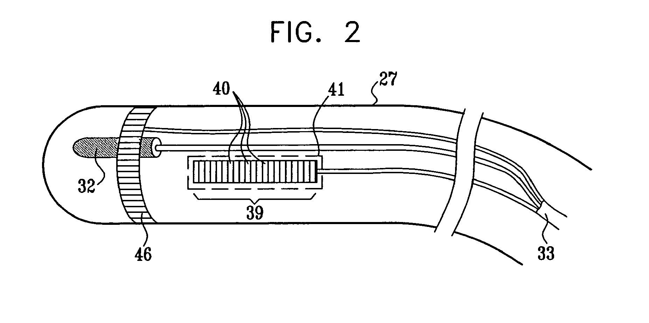

Reference is now made to Fig. 2, which is a pictorial illustration of distal end 29 of catheter 27 used in the system shown in Fig. 1, in accordance with an embodiment of the present invention. The generated fields described above are sensed by a position sensor 32 located within distal end 29 of catheter 27.

Position sensor 32 transmits, in response to the sensed fields, position-related electrical signals over cables 33 running through catheter 27 to a console 34 (Fig. 1). Alternatively, position sensor 32 may transmit signals to the console over a wireless link.

In an alternate embodiment, one or more radiators in the catheter, typically coils, generate magnetic fields which are received by sensors outside the patient's body. The external sensors generate the position-related electrical signals.

Referring again to Fig. 1, console 34 comprises a positioning processor 36 that calculates the location and orientation of distal end 29 of catheter 27 based on the signals sent by position sensor 32 (Fig. 2). Positioning processor 36 typically receives, amplifies, filters, digitizes, and otherwise processes signals from sensor 32.

Some position tracking systems that may be used in embodiments of the present invention are described, for example, in U.S Patent 6,690,963, cited above, as well as in U.S. Patents 6,618,612 and 6,332,089, and U.S. Patent Application Publications 2004/0147920 A1 and 2004/0068178 A1, all of which are incorporated herein by reference. Although positioning subsystem 30 uses magnetic fields, embodiments of the present invention may be implemented using any other suitable positioning subsystem, such as systems based on electromagnetic field measurements, acoustic measurements and/or ultrasonic measurements.

Referring again to Fig. 2, catheter 27 comprises an ultrasonic imaging sensor 39, located within distal end 29. Ultrasonic imaging sensor 39 typically comprises an array of ultrasonic transducers 40. Although ultrasonic transducers 40 are shown arranged in a linear array configuration, other array configurations may be used, such as circular or convex configurations. In one embodiment, ultrasonic transducers 40 are piezo-electric transducers. Ultrasonic transducers 40 are positioned in or adjacent to a window 41, which defines an opening within the body or wall of catheter 27.

Transducers 40 operate as a phased array, jointly transmitting an ultrasound beam from the array aperture through window 41. In one embodiment, the array transmits a short burst of ultrasound energy and then switches to a receiving mode for receiving the ultrasound signals reflected from the surrounding tissue. Typically, transducers 40 are driven individually in a controlled manner in order to steer the ultrasound beam in a desired direction. By appropriate timing of the transducers, the produced ultrasound beam may be given a concentrically curved wave front, so as to focus the beam at a given distance from the transducer array. Typically, system 20 comprises a transmit/receive scanning mechanism that enables steering and focusing of the ultrasound beam, and recording of reflections from the beam, so as to produce 2-D ultrasound images.

In one embodiment, ultrasonic imaging sensor 39 comprises between sixteen and sixty-four ultrasonic transducers 40, typically between forty-eight and sixty-four ultrasonic transducers 40. Typically, ultrasonic transducers 40 generate the ultrasound energy at a center frequency in a range of 5-10 MHz, with a typical penetration depth ranging from several millimeters to around 16 centimeters. The penetration depth depends upon the characteristics of ultrasonic imaging sensor 39, the characteristics of the surrounding tissue, and the operating frequency. In alternative embodiments, other suitable frequency ranges and penetration depths may be used.

Ultrasonic transducers 40 may also detect the frequency of ultrasonic waves received. A change between the transmitted and received frequencies indicates a Doppler shift, which may be used to calculate the component of the velocity, in the direction of the ultrasound beam, of an object that reflects the beam.

A suitable catheter that may be used in system 20 is the SOUNDSTAR^{™} catheter, manufactured and sold by Biosense Webster Inc., 3333 Diamond Canyon Road, Diamond Bar, CA 91765.

Referring again to Fig. 1, after receiving the reflected ultrasound echoes, electric signals based on the reflected echoes are sent by ultrasonic transducers 40 (Fig. 2) over cables 33 through catheter 27 to an image processor 43 in console 34. Processor 43 transforms the signals into 2-D, typically sector-shaped, ultrasound images and corresponding 2-D Doppler images. Image processor 43 typically displays real-time ultrasound images of sections of heart 24, performs 3-D image or volume reconstructions of the sections, and performs other functions described in greater detail below.

In some embodiments, the image processor uses the ultrasound images and the positional information to produce a 3-D model of an anatomical structure such as the patient's heart. In the context of the present patent application and in the claims, the term "anatomical structure" refers to a chamber of an organ such as the heart, in whole or in part, or to a particular wall, surface, blood vessel or other anatomical feature of the heart or other organ. The 3-D model is presented to the physician as a 2-D projection on a display 44.

In some embodiments, distal end 29 of catheter 27 also comprises at least one electrode 46 for performing diagnostic and/or therapeutic functions, such as electro-anatomical mapping and/or radio frequency (RF) ablation. In one embodiment, electrode 46 is used for sensing local electrical potentials. The electrical potentials measured by electrode 46 may be used in mapping the local electrical activity on the endocardial surface. When electrode 46 is brought into contact or proximity with a point on the inner surface of the heart, it measures the local electrical potential at that point. The measured potentials are converted into electrical signals and sent through the catheter to the image processor for display. In other embodiments, the local electrical potentials are obtained from another catheter comprising suitable electrodes and a position sensor, all connected to console 34.

In alternative embodiments, electrode 46 may be used to measure different parameters. For example, electrode 46 may be used to measure various tissue characteristics. Additionally or alternatively, electrode 46 may be used to measure temperature. Further additionally or alternatively, electrode 46 may be used to measure a rate of flow of blood. Although electrode 46 is shown as being a single ring electrode, the catheter may comprise any convenient number of electrodes 46 in forms known in the art. For example, the catheter may comprise two or more ring electrodes, a plurality or array of point electrodes, a tip electrode, or any combination of these types of electrodes for performing the diagnostic and/or therapeutic functions outlined above.

Position sensor 32 is typically located within distal end 29 of catheter 27, adjacent to electrode 46 and transducers 40. Typically, the mutual location and orientation offsets between position sensor 32, electrode 46 and transducers 40 of ultrasonic sensor 39 are constant. These offsets are typically used by positioning processor 36 to derive the coordinates of the ultrasonic sensor and of electrode 46, given the measured position of position sensor 32. In another embodiment, catheter 27 comprises two or more position sensors 32, each having constant location and orientation offsets with respect to electrode 46 and transducers 40. In some embodiments, the offsets (or equivalent calibration parameters) are pre-calibrated and stored in positioning processor 36. Alternatively, the offsets may be stored in a memory device, such as an EPROM (Erasable Programmable Read Only Memory), fitted into handle 28 of catheter 27.

Position sensor 32 typically comprises three non-concentric coils (not shown), such as are described in U.S. Patent 6,690,963 cited above. Alternatively, any other suitable position sensor arrangement may be used, such as sensors comprising any number of concentric or non-concentric coils, Hall-effect sensors and/or magnetoresistive sensors.

Typically, both the ultrasound images derived from sensor 39 and the position measurements of sensor 32 are synchronized with the heart cycle, by gating signal and image captures relative to a body-surface electrocardiogram (ECG) signal or intra-cardiac electrocardiogram. In one embodiment, the ECG signal may be produced by electrode 46. Since features of the heart change their shape and position during the heart's periodic contraction and relaxation, the entire imaging process is typically performed at a particular point in time with respect to this period. In some embodiments, additional measurements taken by the catheter, such as those described above, are also synchronized to the electrocardiogram (ECG) signal. These measurements are also associated with corresponding position measurements taken by position sensor 32. The additional measurements are typically overlaid on the reconstructed 3-D model, as will be explained below.

In some embodiments, the position measurements and the acquisition of the ultrasound images are synchronized to an internally-generated signal produced by system 20 (Fig. 1). For example, a synchronization mechanism may be used to avoid interference in the ultrasound images caused by an internal interfering signal. In this case, the timing of image acquisition and position measurement is set to a particular offset with respect to the interfering signal, so that images are acquired without interference. The offset may be adjusted occasionally to maintain interference-free image acquisition. Alternatively, the measurement and acquisition may be synchronized to an externally-supplied synchronization signal.

In some embodiments image processor 43 may use successive position measurements of position sensor 32 to estimate a speed of movement of distal end 29. Typically, the physician operates apparatus 20 to generate ultrasound images when the speed of movement is below a pre-set threshold, the threshold being set so that providing the movement is below the threshold there is substantially no effect on the measured Doppler shifts, and so on derived velocities of objects producing the shifts. Alternatively or additionally, apparatus may be configured so that a velocity component of distal end 29 in the direction of the ultrasound beam is added to a velocity component, derived from a measured Doppler shift, of an object that reflects the ultrasound beam. The vector addition of the components corrects for the movement of distal end 29.

In one embodiment, system 20 comprises an ultrasound driver (not shown) that drives the ultrasound transducers 40. One example of a suitable ultrasound driver, which may be used for this purpose is an AN2300TM ultrasound system produced by Analogic Corp. of Peabody, Massachusetts. In this embodiment, the ultrasound driver performs some of the functions of image processor 43, driving the ultrasonic sensor and producing the 2-D ultrasound images. The ultrasound driver may support different imaging modes such as B-mode, M-mode (one-dimensional diagnostic ultrasound with time shown on the perpendicular axis), CW (Continuous Wave) Doppler (which uses a continuous wave of ultrasound energy to detect velocity of objects) and color flow Doppler (which uses pulses of ultrasound energy to determine distance as well as velocity of objects, and displays the resulting images using colors according to relative velocity), as are known in the art.

Typically, the positioning and image processors are implemented using a general-purpose computer, which is programmed in software to carry out the functions described herein. The software may be downloaded to the computer in electronic form, e.g. over a network, or it may alternatively be supplied to the computer on tangible media, such as CD-ROM. The positioning processor and image processor may be implemented using separate computers or using a single computer, or may be integrated with other computing functions of system 20. Additionally or alternatively, at least some of the positioning and image processing functions may be performed using dedicated hardware.

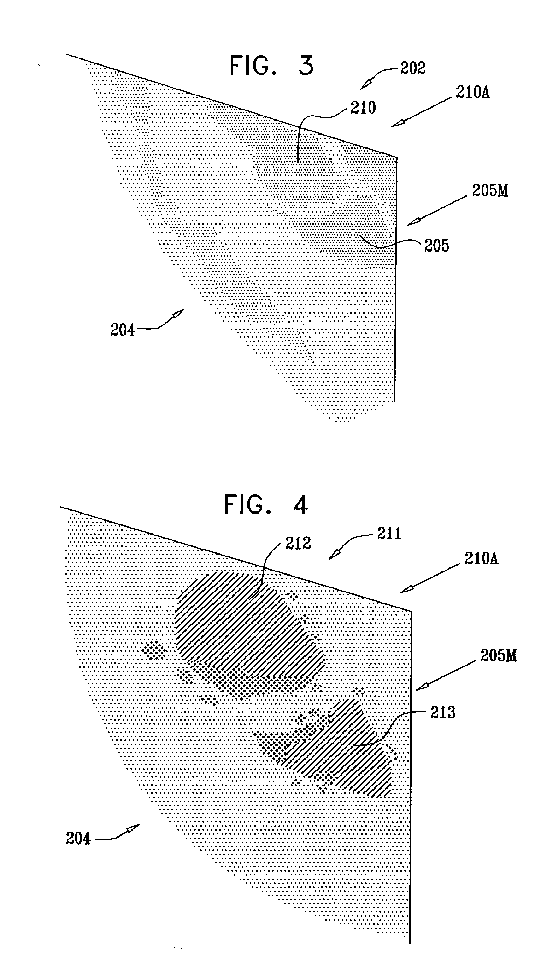

Reference is now made to Figs. 3, 4, 5 and 6, which are schematic images of a non-human heart, in accordance with an embodiment of the present invention.
Fig. 3 illustrates a 2-D ultrasound image 202 of a part of a non-human heart. The image was taken with the catheter positioned in the right atrium of a heart 204 of a pig, and shows a feature 205, which represents the ultrasound intensities generated by objects in the vicinity of a mitral valve 205M, and a feature 210, which represents the ultrasound intensities generated by objects in the vicinity of an aortic valve 210A. Although features 205, 210 are shown in Fig. 3, their boundaries are not clearly delineated. Typically, a corresponding 2-D image of human heart 24 may be displayed to the physician on display 44. The images generated on display 44, of heart 204 or of heart 24, are typically in color. Different intensities of the images on display 44 are represented in Fig. 3 by different shadings.

Fig. 4 illustrates a 2-D Doppler image 211 of the part of heart 204 shown in 2-D ultrasound image 202 (Fig. 4). 2-D Doppler image 211 is an ultrasonic image containing Doppler information, typically generated by blood flow, in the vicinity of mitral valve 205M and aortic valve 210A. A feature 212 shows movement in the vicinity of aortic valve 210A, a feature 213 shows movement in the vicinity of mitral valve 205M. Movement in the direction of the ultrasound beam is typically shown by different colors. For example, movement away from ultrasonic imaging sensor 39 (Fig. 2) may appear as red on display 44, and movement towards ultrasonic imaging sensor 39 may appear as blue on display 44. Different colors of the images on display 44 are represented in Fig. 4 by different shadings, wherein diagonal stripes represent speeds between approximately +0.2 m/s and +0.6 m/s, small dots represent speeds between approximately -0.2 m/s and +0.2 m/s, and large dots represent speeds between approximately -0.6 m/s and -0.2 m/s. A positive speed indicates movement away from sensor 39 and a negative speed indicates movement towards the sensor.

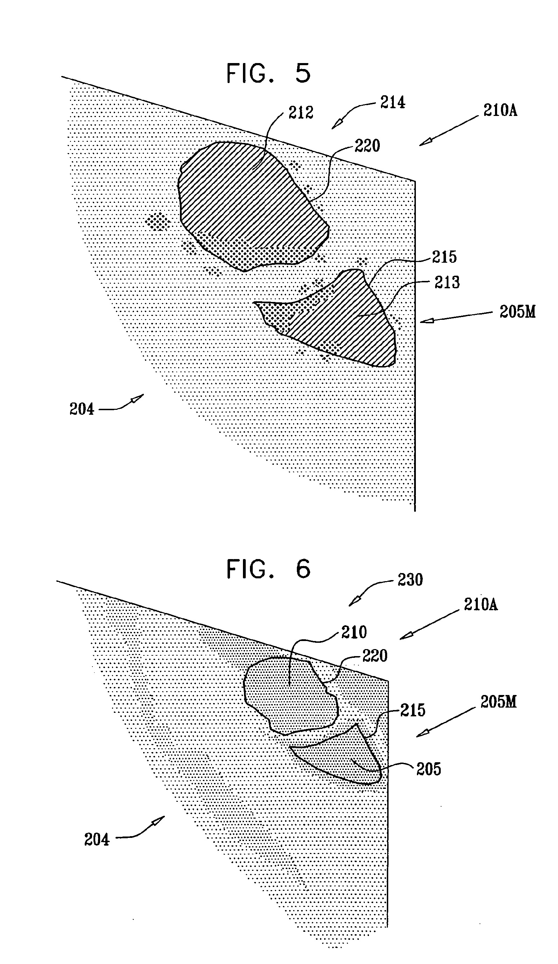

Fig. 5 illustrates an enhanced version 214 of 2-D Doppler image 211 showing contours derived from the Doppler information. The contours may be derived by an image processor such as processor 43 determining boundaries between areas of rapid movement, e.g. having speeds more than 0.2 m/s, which typically represent flow of blood, and areas of little or no movement, e.g. having speeds less than 0.03 m/s. Since, compared to the speed of blood flow, speeds of movement of heart chamber walls and/or blood vessels are typically small, the contours typically represent the internal walls of the heart chambers and blood vessels. Feature 213 has been marked with a contour 215. Feature 212 has been marked with a contour 220.

Fig. 6 is an enhanced version 230 of 2-D ultrasound image 202 (Fig. 3). Contours 215 and 220, derived from the Doppler information, have been mapped onto the 2-D ultrasound intensity image. Fig. 5 and Fig. 6 demonstrate that by displaying the contours on the ultrasound intensity image or on the Doppler information image, the physician may more accurately, and more easily, perceive the boundaries of aortic valve 210A and mitral valve 205M.

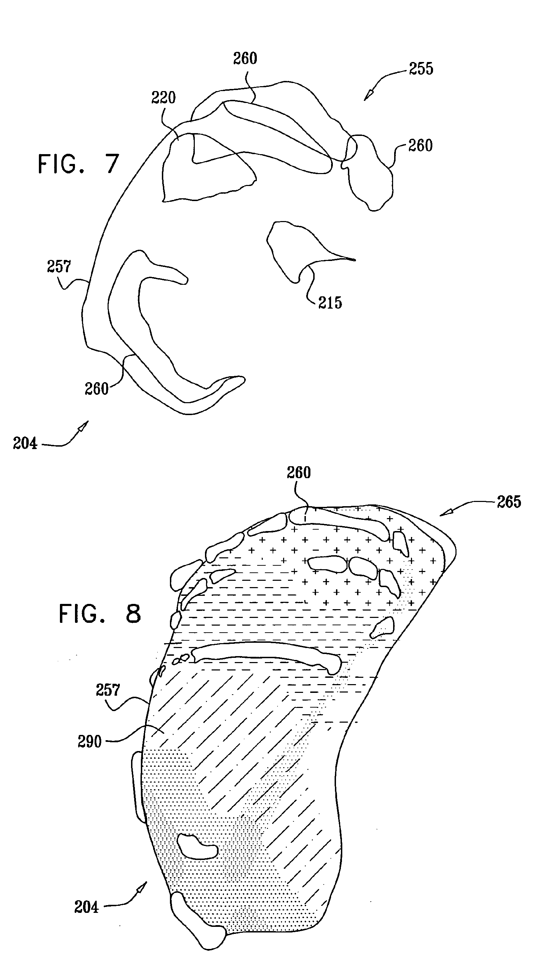

Reference is now made to Fig. 7, which is a 3-D skeleton model 255 of a left ventricle 257 of heart 204, in accordance with an embodiment of the present invention. The skeleton model comprises a plurality of contours in 3-D space. 3-D skeleton model 255 shows contours 215 and 220 from a different viewpoint to that of Fig. 6. 3-D skeleton model 255 also shows additional contours 260, derived in the same manner as contours 215 and 220, using 2-D Doppler ultrasonic images obtained from other positions of ultrasonic imaging sensor 39. For clarity, only a few contours are shown in Fig. 7.

Reference is now made to Fig. 8, which is a 3-D surface model 265 of left ventricle 257, in accordance with an embodiment of the present invention. Model 265 is obtained using a "wire-mesh" type process, in which 3-D skeleton model 255, including additional contours not shown in Fig. 7, is virtually encased to generate surfaces over the skeleton model and produce a 3-D shape of the anatomical structure. The generated surface of left ventricle 257 is overlaid with an electrical activity map 290, as described hereinbelow. The map presents different electrical potential values using different colors (shown as different shading patterns in Fig. 8).

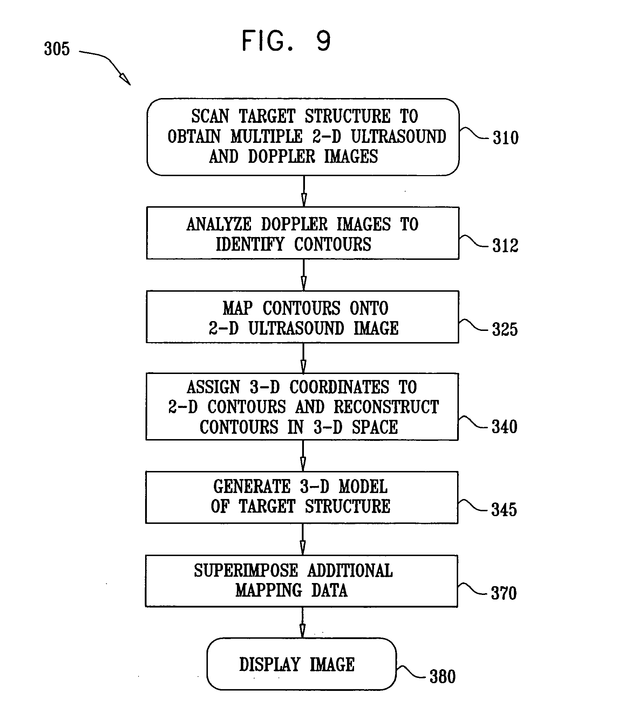

Reference is now made to Fig. 9, which is a flow chart 305 that schematically illustrates a method for cardiac mapping and imaging, in accordance with an embodiment of the present invention. The method of flow chart 305 typically combines multiple 2-D ultrasound images, acquired at different positions of ultrasonic imaging sensor 39 (Fig. 2), into a single 3-D model of the anatomical structure.

In an initial step 310, a sequence of 2-D ultrasound images of the anatomical structure is acquired. Typically, the physician inserts catheter 27 through a suitable blood vessel into a chamber of heart 24, such as the right atrium, and then scans the anatomical structure by moving the distal end of the catheter between different positions inside the chamber. The anatomical structure may comprise all or a part of the chamber in which the catheter is located or, additionally or alternatively, a different chamber, such as the left atrium, or vascular structures, such as the aorta. In each position of ultrasonic imaging sensor 39, the image processor acquires and produces a 2-D ultrasound intensity image and, typically, a 2-D ultrasound Doppler image, using signals received from ultrasonic imaging sensor 39.

In parallel, the positioning sub-system measures and calculates the position of the distal end of the catheter. The calculated position is stored together with the corresponding ultrasound image. Typically, each position of the distal end of the catheter is represented in coordinate form, such as a six-dimensional coordinate (X, Y, Z axis positions and pitch, yaw and roll angular orientations).

In a step 312, the image processor analyzes each 2-D Doppler image 211 to identify contours of entities, as described above for Fig. 5.

In a step 325, contours are mapped onto each 2-D ultrasound image, as illustrated in Fig. 6, described above. The contours mark boundaries of the anatomical structures in the 3-D working volume and assist the physician to identify these structures during the procedure.

Steps 312 and 325 are performed for all 2-D ultrasound images produced at step 310. In some cases, where image processor 43 (Fig. 1) is unable to deduce the location of part of a contour from the corresponding 2-D Doppler image, the processor may use the contours derived from other 2-D ultrasound and Doppler images, typically images spatially adjacent to the image in question, to automatically identify and reconstruct the contour. This identification and reconstruction process may use any suitable image processing method, including edge detection methods, correlation methods and other methods known in the art. The image processor may also use the position coordinates of the catheter that are associated with each of the images in correlating the contour locations from image to image. Additionally or alternatively, step 312 may be implemented in a user-assisted manner, in which the physician reviews and corrects the automatic contour reconstruction carried out by the image processor, using either the 2-D ultrasound image or the 2-D Doppler image, or both images.

In a step 340, the image processor assigns 3-D coordinates to the contours identified in the set of images. The location and orientation of the planes of the 2-D ultrasound images in 3-D space are known by virtue of the positional information, stored together with the images at step 310. Therefore, the image processor is able to determine the 3-D coordinates of any pixel in the 2-D images, and in particular those corresponding to the contours. When assigning the coordinates, the image processor typically uses the stored calibration data comprising the location and orientation offsets between the position sensor and the ultrasonic sensor, as described above.

In a step 345, the image processor produces a 3-D skeleton model of the anatomical structure, as described above for Fig. 7. In some embodiments, the image processor produces a 3-D surface model, such as image 265 (Fig. 8), by virtually encasing the 3-D skeleton model as described above.

As described above, in some embodiments system 20 (Fig. 1) supports a measurement of local electrical potentials on the surfaces of the anatomical structure. Each electrical activity data-point acquired by catheter 27 (Fig. 2) comprises an electrical potential or activation time value measured by electrode 46 (Fig. 2) and the corresponding position coordinates of the catheter measured by the positioning sub-system. In a step 370, the image processor registers the electrical activity data-points with the coordinate system of the 3-D model and overlays them on the model. This is shown as electrical activity map 290 in Fig. 8. Step 370 is optional in the method and is performed only if system 20 supports this type of measurement and if the physician has chosen to use this feature.

Alternatively, a separate 3-D electrical activity map (often referred to as an electro-anatomical map) may be generated and displayed. For example, a suitable electro-anatomical map may be produced by a CARTO^{™} navigation and mapping system, manufactured and sold by Biosense Webster, Inc. The electrical potential values may be presented using a color scale, for example, or any other suitable visualization method. In some embodiments, the image processor may interpolate or extrapolate the measured electrical potential values and display a full color map that describes the potential distribution across the walls of the anatomical structure.

As noted above, information imported from other imaging applications may be registered with the 3-D model and overlaid on the model for display. For example, pre-acquired computerized tomography (CT), magnetic resonance imaging (MRI) or x-ray information may be registered with the 3-D ultrasound-based model.

Additionally or alternatively, if additional measurements were obtained using electrode 46 as described above, these measurements may be registered with the 3-D model and displayed as an additional layer, often referred to as a parametric map.

In a final step 380, the 3-D model is typically presented to the physician on display 44 (Fig. 1).

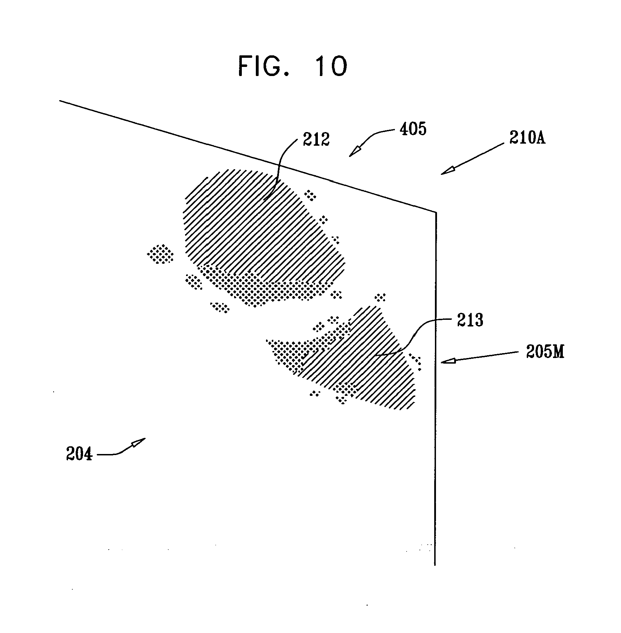

Reference is now made to Fig. 10, which is a schematic image of a non-human heart, in accordance with an alternate embodiment of the present invention. Fig. 10 illustrates a 2-D Doppler image 405 of heart 204. Apart from the differences described below, image 405 is generally similar to images 211 and 214 (Figs. 4 and 5), and elements indicated by the same reference numerals in images 405, 211, and 214 have generally similar descriptions. In 2-D Doppler image 405 only areas of movement are shown. Thus, features 212, 213 are shown, representing movement in vicinity of the aortic valve and mitral valve respectively, as in Figs. 4 and 5. However, in image 405, a threshold is set at 0.08 m/s so that objects having derived speeds between -0.08 m/s and +0.08 m/s are not displayed. Thus, in contrast to images 211 and 214, in image 405 no contours nor regions that have slow derived speeds are displayed.

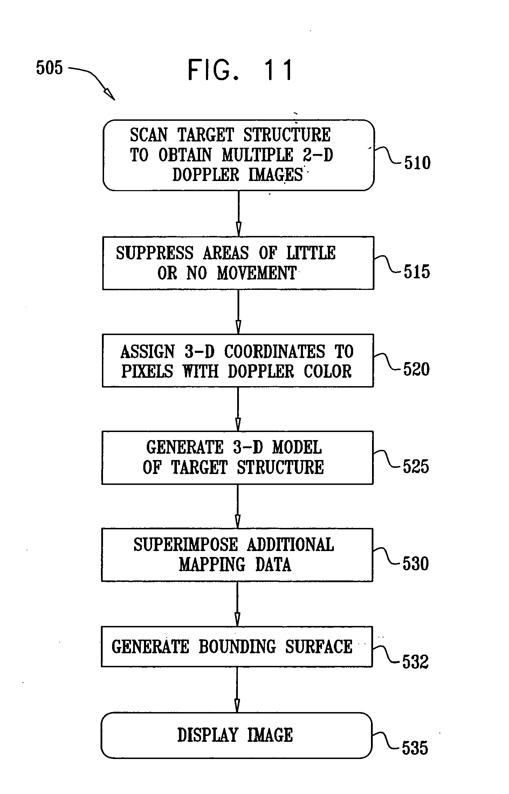

Reference is now made to Fig. 11, which is a flow chart 505 that schematically illustrates a method for cardiac mapping and imaging, in accordance with an alternate embodiment of the present invention. The method of flow chart 505 typically combines multiple 2-D Doppler images, acquired at different positions of ultrasonic imaging sensor 39 (Fig. 2), into a 3-D model of the objects generating the images.

An initial step 510 is generally similar to step 310 (Fig. 9). In step 510 a sequence of 2-D Doppler images of the anatomical structure, including elements moving in proximity to the structure is acquired. The moving elements typically comprise a fluid such as blood. In step 510 the positioning sub-system measures and calculates the position of the distal end of the catheter.

In a step 515, the image processor analyzes each 2-D Doppler image 211 to identify areas of movement. Areas of little or no movement are suppressed as described above for Fig. 10. Typically, a pixel is shown only if the speed at the location of the pixel, in the direction of the ultrasound beam, exceeds a threshold. In the case of 2-D Doppler image 405 (Fig. 10), the threshold may be approximately 0.08 m/s.

In a step 520, the image processor assigns 3-D coordinates to the remaining pixels, typically colored, in the set of 2-D Doppler images. The location and orientation of the planes of the 2-D ultrasound images in 3-D space are known by virtue of the positional information, stored together with the images at initial step 510. Therefore, the image processor is able to determine the 3-D coordinates of any pixel in the 2-D images. When assigning the coordinates, the image processor typically uses the stored calibration data comprising the location and orientation offsets between the position sensor and the ultrasonic sensor, as described above.

In a step 525, the image processor produces a 3-D image comprising all the pixels, in 3-D space, of points of movement in proximity to the anatomical structure.

In an optional step 530, additional data may be superimposed on the 3-D image, as described above for step 370 of flow chart 305 (Fig. 9).

In a further optional step 532, the image processor may generate a bounding surface around pixels produced in step 525. To generate the bounding surface, the image processor may perform an iterative process to determine the surface. For example, the processor or the physician may select a seed point from which to begin generating the surface. The processor iteratively finds the surface by radiating from the point until all pixels above a predefined threshold, such as the threshold of step 515, have been identified. The processor determines the surface enclosing the identified pixels. Alternatively, the processor may use all pixels identified by radiating from the seed point, regardless of threshold, to generate the bounding surface.

In a final step 535, the image generated in the preceding steps is typically presented to the physician on display 44 (Fig. 1). It will be appreciated that implementation of flowchart 505 enables the physician to see a map or a model of movement of elements moving in proximity to 3-D anatomical structures, such as blood that is flowing. Alternatively or additionally, the physician is able to see a bounding surface related to the moving elements.

In some embodiments, system 20 (Fig. 1) may be used as a real-time or near real-time imaging system. For example, the physician may reconstruct a 3-D model of an anatomical structure, and/or of objects moving in proximity to an anatomical structure, using the methods described above, as a preparatory step before beginning a medical procedure. During the procedure, system 20 may continuously track and display the 3-D position of the catheter with respect to the model. The catheter used for performing the medical procedure may be the same catheter used for generating the 3-D model, or a different catheter fitted with a suitable position sensor.

Although the embodiments described above relate to ultrasound imaging using an invasive probe, such as a cardiac catheter, the principles of the present invention may also be applied in reconstructing 3-D models of organs using an external or internal ultrasound probe (such as a trans-thoracic probe), fitted with a positioning sensor. Additionally or alternatively, as noted above, the disclosed methods may be used for 3-D modeling of organs other than the heart, for example blood vessels leading into and out of the heart chambers, or organs such as the carotid artery. Further additionally or alternatively, other diagnostic or treatment information, such as tissue thickness and ablation temperature, may be overlaid on the 3-D model in the manner of the electrical activity overlay described above. The 3-D model may also be used in conjunction with other diagnostic or surgical procedures, such as ablation catheters.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art.

## Claims

1. A method for imaging an anatomical structure, comprising:
acquiring a plurality of ultrasonic images of the anatomical structure, at least one of the images comprising Doppler information;
generating one or more contours of the anatomical structure using the Doppler information; and
reconstructing a three-dimensional image of the anatomical structure from the plurality of ultrasonic images using the one or more contours.

2. The method according to claim 1, wherein generating the one or more contours comprises determining a boundary between a first region of the anatomical structure having a speed of movement greater than or equal to a first value and a second region of the anatomical structure wherein the speed of movement is less than or equal to a second value smaller than the first value.

3. The method according to claim 2, wherein the first value is 0.08 m/s and the second value is 0.03 m/s.

4. The method according to claim 1, wherein the anatomical structure comprises a heart, and wherein acquiring the plurality of ultrasonic images comprises inserting a catheter comprising an ultrasonic sensor into a chamber of the heart and moving the catheter between a plurality of spatial positions within the chamber.

5. The method according to claim 4, further comprising measuring location and orientation coordinates of the ultrasonic sensor, and synchronizing the plurality of ultrasonic images and the location and orientation coordinates relative to a synchronizing signal comprising one of an electrocardiogram (ECG) signal, an internally-generated synchronization signal and an externally-supplied synchronization signal.

6. The method according to claim 5, wherein the three-dimensional image comprises a three-dimensional surface model of the anatomical structure, further comprising:
measuring at least one of a tissue characteristic, a temperature and a rate of flow of blood, synchronized to the synchronizing signal, to produce a parametric map; and
overlaying the parametric map on the three-dimensional surface model.

7. The method according to claim 1, wherein acquiring the plurality of ultrasonic images comprises moving an ultrasonic sensor generating the ultrasonic images so that a velocity of movement of the ultrasonic sensor is less than a pre-determined threshold velocity.

8. The method according to claim 1, wherein acquiring the plurality of ultrasonic images comprises determining a velocity of movement of an ultrasonic sensor generating the ultrasonic images, and correcting the Doppler information responsively to the velocity of movement.

9. The method according to claim 1, wherein the three-dimensional image comprises a three-dimensional skeleton model of the anatomical structure.

10. The method according to claim 1, wherein the three-dimensional image comprises a three-dimensional surface model of the anatomical structure.

11. The method according to claim 10, further comprising overlaying an electro-anatomical map on the three-dimensional surface model.

12. The method according to claim 10, further comprising overlaying information imported from one or more of a Magnetic Resonance Imaging (MRI) system, a Computerized Tomography (CT) system and an x-ray imaging system on the three-dimensional surface model.

13. A method for imaging an anatomical structure, comprising:
acquiring a plurality of two-dimensional Doppler images of elements moving in proximity to the anatomical structure; and
reconstructing a three-dimensional image of the moving elements.

14. The method according to claim 13, wherein reconstructing the three-dimensional image comprises displaying the three-dimensional image absent the anatomical structure.

15. The method according to claim 13, and comprising setting a threshold speed for the moving elements, and wherein reconstructing the three-dimensional image comprises displaying the moving elements having speeds greater than the threshold speed.

16. The method according to claim 13, wherein reconstructing the three-dimensional image comprises determining a surface bounding at least some of the elements, and displaying the surface.

17. A system for imaging an anatomical structure, comprising:
a probe, comprising an ultrasonic sensor, which is configured to acquire a plurality of ultrasonic images of the anatomical structure, at least one of the images comprising Doppler information; and
a processor, coupled to the ultrasonic sensor, which is configured to generate one or more contours of the anatomical structure using the Doppler information and to reconstruct a three-dimensional image of the anatomical structure from the plurality of ultrasonic images using the one or more contours.

18. A system for imaging an anatomical structure, comprising:
a probe, comprising an ultrasonic sensor, which is configured to acquire a plurality of two-dimensional Doppler images of elements moving in proximity to the anatomical structure; and
a processor which is configured to reconstruct a three-dimensional image of the moving elements from the two-dimensional Doppler images.

19. A computer software product for imaging an anatomical structure, comprising a computer-readable medium in which computer program instructions are stored, which instructions, when read by a computer, cause the computer to acquire a plurality of ultrasonic images of the anatomical structure, at least one of the images comprising Doppler information, to generate one or more contours of the anatomical structure using the Doppler information, and to reconstruct a three-dimensional image of the anatomical structure from the plurality of ultrasonic images using the one or more contours.
